# EUROPEAN PATENT SPECIFICATION

(11) **EP 0 662 611 B1**
(45) Date of publication and mention of the grant of the patent: **06.12.2000**
(21) Application number: 94115865.1
(22) Date of filing: 07.10.1994
(51) Int. Cl.: G01N 33/543, G01N 33/564, G01N 33/58, G01N 33/92

(54) **Method for assaying a rheumatoid factor and kit thereof**
Verfahren zur Bestimmung vom rheumatoiden Faktor und Kit dazu
Méthode pour l'essai de facteur rhumatoide et une trousse de ceci

(30) Priority: 19.11.1993 JP 29053793
(43) Date of publication of application: 12.07.1995
(73) Proprietor: Eisai Co., Ltd., Tokyo (JP)
(72) Inventor: Yamada, Yuji, Tsukuba-shi, Ibaraki (JP)
(74) Representative: Thiel, Christian, Dr. Dipl.-Chem.

(56) References cited:
- EP-A- 0 398 292
- WO-A-89/04490
- PATENT ABSTRACTS OF JAPAN vol. 013, no. 146 (P-854), 11 April 1989 & JP-A-63 308561 (NAOYUKI TANIGUCHI), 15 December 1988,
- DATABASE WPI Week 9115 Derwent Publications Ltd., London, GB; AN 91-106293 XP002005623 & JP-A-03 048 700 (G H FUJITA GAKUEN; EISAI KK) , 1 March 1991

## Description

### Background of the Invention

### Field of the Invention

The present invention relates to a method for assaying a rheumatoid factor with the use of lectins and a kit therefor.

### Description of the Related Art

Glycoconjugate is a generic name representing compounds having a sugar chain, such as a glycoprotein and a glycolipid. First, a rheumatoid factor, which is a glycoprotein, will be described as an example of glycoconjugates.

It is known that an autoimmune antibody called a rheumatoid factor, which recognizes an epitope located at the Fc site of human immunoglobulin G (hereinafter referred to simply as IgG) as an antigen, is present in the serum of a patient with rheumatoid arthritis (hereinafter referred to simply as an RA patient) [see H.G. Kunkel et al., Adv. Immunol., 4, 351 (1964)]. Namely, the rheumatoid factor is an autoantibody against IgG which is an autologous immunoglobulin and can be classified into IgA, IgG and IgM types depending on the varieties.

Assaying a rheumatoid factor, which is one of important pathogeneses of RA, is an effective means of diagnosing RA and several methods including the determination of the rheumatoid factor have been employed for diagnosing RA. Typical examples thereof include a method which comprises reacting a serum specimen with a modified IgG bound to latex beads and determining the presence of a rheumatoid factor based on the agglutination of the latex beads [see Singer, J.M., et al., Am. J. Med., 21, 888 (1956)] and an enzyme immunoassay method which comprises binding a rheumatoid factor contained in a specimen with IgG or IgGFc which has been immobilized on a solid phase and binding an enzyme-labeled antibody as the second antibody capable of recognizing the Fab site of immunoglobulin, i.e., the rheumatoid factor, as the antigen (see GB 2001171A).

The method with the use of the agglutination is disadvantageous that rheumatoid factors of various types other than IgM type, such as IgA and IgG, cannot be assayed thereby, though a rheumatoid factor of the IgM type can be assayed thereby. Further, it is disadvantageous in poor assay accuracy. In the enzyme immunoassay method, a false positive ratio of several % is given even in healthy subjects and, furthermore, a high false positive ratio is observed in those suffering from liver diseases, autoimmune diseases other than rheumatoid arthritis and osteoarthritis, which makes it necessary to carry out a confirmation test by using another technique. In addition, a number of patients with rheumatoid arthritis are false negative in this assay. Thus this method is not satisfactory in reliability.

Recently, there has been reported a method for assaying a rheumatoid factor by utilizing the sugar chain of a rheumatoid factor without employing any second antibody. Specifically, this method comprises assaying a rheumatoid factor, which is an autoantibody, i.e., immunoglobulin, and a glycoprotein, with the use of a lectin having an avidity for the sugar chain of the rheumatoid factor (see Japanese Patent Publication-A No. 3-48700, published on March 3, 1991). The result of the analysis on the sugar chain of IgG, i.e., a rheumatoid factor, contained in bloods of RA patients has revealed deficiency of galactose therein, compared with normal IgG. Based on this finding, this method has been developed. Namely, in this method, the rheumatoid factor is assayed based on the difference between the binding activity of a lectin to the galactose-deficient sugar chain and that to the normal sugar chain. Further, European Patent Publication-A No. 0398292 (published on Nov. 22, 1990) discloses a diagnostic drug for rheumatoid arthritis comprising a lectin and a galactose-deficient IgG. However, this method and this diagnostic drug have a disadvantage that the lectin usable therefor is restricted, since the difference between the binding activities is utilized as described above. Further, they are not established but should be improved in future from the viewpoints of the troublesome procedure for the construction of the galactose-deficient IgG which is used for immobilization, and the economic disadvantage.

Accordingly, it has been required to improve the above-mentioned method for assaying a rheumatoid factor with the use of a sugar chain of a rheumatoid factor to thereby develop a highly specific method for assaying a rheumatoid factor at a high accuracy.

The document JP63308561 discloses a method for assaying an antigen with a glycochain structure using as a first reactant a modified antibody which has been treated to diminish the binding with a sugar chain-binding substance, while maintaining the reactivity to the antigen.

An object of the present invention is to provide a method for assaying a rheumatoid factor at a high disease specificity and a high accuracy and a kit therefor.

### Disclosure of the Invention

### Summary of the Invention

The present inventors have attempted to improve the method for assaying a rheumatoid factor with the use of a lectin.

To trap a rheumatoid factor contained in a specimen, there has been commonly used IgG or IgGFc which has been immobilized on a solid phase in consideration of the characteristics of a rheumatoid factor. A method which comprises using IgG or IgGFc which has been immobilized on a solid phase is the most desirable therefor. However, when a trapped rheumatoid factor is assayed with the use of a lectin, the method has a serious problem that the lectin reacts with the immobilized IgG or IgGFc. Accordingly, the present inventors have considered that an excellent rheumatoid factor assay method improved in the sensitivity and accuracy might be established by using a modified IgG which has been prepared by chemically treating a sugar chain of IgG to thereby cause the loss of its avidity for a lectin while maintaining its avidity for a rheumatoid factor.

It is known that hydroxyl groups of a saccharide play an important role in the avidity of the saccharide for a lectin. Therefore, the avidity of a modified IgG, which had been prepared by treating the hydroxyl groups of the sugar chain(s) in normal IgG with periodic acid and of which avidity for lectins had been lost, for a rheumatoid factor was examined. As a result, it was found for the first time that the modified IgG, i.e., the periodic acid-treated IgG, maintained the avidity for a rheumatoid factor similar to normal IgG. As the results of extensive studies with the use of this periodic acid-treated IgG, the present inventors have established an objective excellent method for assaying a rheumatoid factor. Further, they have found that the method is applicable as an assay method for glycoconjugates in general. The present invention has been completed on the basis of these findings.

Thus, the present invention relates to a method for assaying a RF by the sandwich method which comprises:
1) reacting a periodic acid-treated immunoglobulin (IgG), which has been prepared by treating a sugar chain of an IgG having an avidity specific for a RF to thereby cause the loss of its avidity for a lectin, with a specimen to thereby bind the RF in the specimen thereto;
2) reacting said lectin having an avidity for the sugar chain of the RF as a second reactant with the RF thus bound; and
3) determining the amount of the lectin thus bound.

The present invention is characterized in that a substance (A) capable of binding specifically to the rheumatoid factor to be assayed is not used as the first reactant as such but treated to thereby modify the sugar chain of the substance (A) and the resulting modified substance (A), of which avidity for lectin has been lost, i.e., a so-called modified first reactant, is used. When the modified substance (A) is used as the first reactant, lectin employed as the second reactant binds exclusively to the rheumatoid factor to be assayed. As a result, the assay accuracy of the rheumatoid factor can be elevated and highly reliable data can be obtained.

Since the present inventors have found for the first time that when the substance (A) is treated under appropriate conditions, the avidity for the target rheumatoid factor cannot be lost even though its sugar chain is modified, the present invention has been completed.

Further scope and applicability of the present invention will become apparent from the detailed description given hereinafter. However, it should be understood that the detailed description and specific examples, while indicating preferred embodiments of the invention, are given by way of illustration only, since various changes and modifications will become apparent to those skilled in the art from this detailed description.

### Detailed Description of the Invention

Now, the present invention will be described in greater detail by reference mainly to a method for assaying a rheumatoid factor, which is a glycoprotein, as an example of the glycoconjugates.

IgG is used as a substance capable of binding specifically to a rheumatoid factor. The term "modified IgG (including its Fc fragment, etc.) having no avidity for lectins" means an IgG of which avidity for lectins has been lost or has disappeared by chemically, biologically or physically treating IgG.

Examples of the treating method include a method which comprises partly or completely decomposing the sugar chain of IgG with the use of, for example, a saccharolytic enzyme, a method which comprises oxidizing hydroxyl groups of the sugar chain of IgG which play an important role in the avidity for lectins and a method which comprises binding an organic or inorganic group to the sugar chain of IgG. Further, genetic recombination techniques are also involved therein as another treatment methods. More precisely, when the expression of IgG is effected under such conditions as to prevent the addition of a sugar chain in the production of IgG by the genetic recombination technique, a sugar-chain-free IgG or an IgG incapable of reacting with a lectin can be obtained. Any treating method is involved in the scope of the present invention, so long as an IgG of which avidity for lectins has disappeared can be obtained thereby. Preferable examples of the treating method include the periodic acid treatment whereby hydroxyl groups of the sugar chain are oxidized and the treatment with a saccharolytic enzyme. As a matter of course, it is important that the modified IgG obtained by treating IgG maintains, i.e., do not lose, its avidity for a rheumatoid factor. A modified IgG of which avidity for lectins has not completely disappeared but slightly remains is usable in practice and the present invention involves such a case. However, it is needless to say that a modified IgG which loses or disappears its avidity for lectins completely is preferable.

The periodic acid treatment of IgG can be effected by a conventional manner. That is, a modified IgG can be prepared by adding IgG to 25 mM periodic acid solution prepared with PBS (pH 7.4), reacting the IgG therein at room temperature for 1 hour and dialyzing the reaction mixture. The treatment of IgG with a saccharolytic enzyme can be effected by treating IgG with, for example, sialidase, mannosidase, N-acetylglucosaminidase or galactosidase either alone or as a mixture thereof.

The modified IgG of which avidity for lectins has disappeared may be applied to an assay system or a reagent system as such. However, it is preferable from the viewpoint of the convenience to immobilize it onto a solid phase prior to use. The solid phase onto which the modified IgG is immobilized may be arbitrarily selected from among synthetic resin beads, such as polystyrene beads, polyethylene beads and silicone beads, glass beads, magnetic grains, erythrocytes, titer plates and nitrocellulose membranes. Among them, titer plates and polystyrene beads are preferable.

It is possible to use not the whole modified IgG but a part thereof, such as the Fc site thereof. It is needless to say that the modified IgG in the present invention also involves such parts thereof.

A rheumatoid factor can be assayed by determining the amount of the lectin bound to the trapped rheumatoid factor. To determine the amount of the lectin bound to the rheumatoid factor, any method for the determination of lectins, for example, use of a labeled lectin, use of antilectin antibody and use of a reagent capable of binding to lectins may be selected.

In the case of the labeled lectin, any labeling agent or marker, such as an enzyme, a fluorescent substance, a pigment, a chemiluminescent substance, an electroluminescent substance, a metal and an isotope may be used, so long as they can be determined. These labeling agents may be directly bound to the lectin. Alternatively, they may be indirectly bound thereto via, for example, biotin or avidin. Examples of the labeling enzymes include alkaline phosphatase, glucose oxidase, peroxidase and β-galactosidase, and peroxidase is particularly preferable. Examples of the chemi- and electroluminescent substances include lanthanide metal compounds.

The sugar-chain-binding substance to be used as the second reactant is not particularly restricted, so long as it has an avidity for the sugar chain of a rheumatoid factor. Examples of the sugar-chain-binding substance include natural substances, organic compounds and inorganic compounds. Particular examples thereof include lectins, antibodies against sugar chains and boric acid derivatives. Among these substances, lectins and antibodies against sugar chains are preferable and lectins are still more preferable.

As the lectin, any one may be used in the present invention, so long as it has an avidity for a sugar chain. Examples thereof include concanavalin A (Con A), Ricinus communis agglutinin (RCA120), Lens culinaris (LCA), Badeiraea simplicifolia (BSII), Phytolacca americana, Lycopersicon esculentum, Dolichos biflorus and Phaseolus vulgaris (PHA-E). For the determination of a rheumatoid factor, Con A, RCA120 and LCA are preferably used.

The rheumatoid-factor assay method according to the present invention may be effected, for example, in the following manner. The total assay system comprises a solid phase, periodic acid-treated IgG for coating the solid phase, a standard rheumatoid factor or a serum specimen, RCA120-biotin, peroxidase-avidin and a substrate of peroxidase. As the solid phase, a microtiter plate is used. Prior to the assay, the periodic acid-treated IgG is dissolved in a carbonate buffer (pH 8.5), the carbonate buffer containing the periodic acid-treated IgG is pipetted into the wells of the microtiter plate and the microtiter plate is allowed to stand at 4°C overnight. Thus, the surface of the solid phase is coated with the periodic acid-treated IgG. The uncoated part of the surface of the microtiter plate is coated with bovine serum albumin by dissolving bovine serum albumin in a phosphate buffer, pipetting the buffer into the wells and allowing to stand the microtiter plate in the same manner as the one described above.

The assay of the rheumatoid factor according to the present invention may be carried out in accordance with a usual procedure. For example, it may be effected by adding a standard rheumatoid factor or a serum specimen to coated wells and incubating therein, further adding RCA120-biotin and incubating therein, adding peroxidase-avidin and the substrate and incubating therein, ceasing the reaction and then determining the amount of the decomposed substrate with the use of a spectrophotometer, as will be shown in the following Examples.

Now, a specific embodiment of the rheumatoid-factor-assay-reagent will be described as an example of the present invention. It is a kit containing a combination of periodic acid-treated IgG, which is optionally accompanied by a microtiter plate for immobilization, a control sample positive to a rheumatoid factor and another one negative thereto, RCA120, avidin, biotin, peroxidase and a substrate. In this kit, the microtiter plate may be provided in a state of being coated with the periodic acid-treated IgG, and/or a pair of biotin and RCA120 and a pair of the enzyme and avidin may be provided each in a conjugated state. These cases are also involved in the embodiment of the assay reagent of the present invention.

It is needless to say that when a labeling agent other than an enzyme is used, the constitution of the reagent is to be varied depending on the characteristics of each labeling agent. Further, the kit may arbitrarily contain an appropriate standard diluent, a reaction diluent, a liquid for dissolving the substrate, a reaction stopper, etc., for the convenience of the operation of the assay. The present invention is not restricted thereby.

One embodiment of the present invention relates to a novel method for assaying a rheumatoid factor by using a modified IgG having no avidity for lectins and utilizing the reactivity of a lectin with sugar chain. It is a convenient method corresponding to the two-antibody sandwich method. According to the present invention, a method for assaying a rheumatoid factor having a high specificity and a high sensitivity can be provided. A modified IgG having no avidity for lectins is used in none of the conventional systems for assaying a rheumatoid factor. The method of the present invention exerts such an excellent effect that it can detect patients with rheumatoid arthritis at a high specificity. Thus, it is highly useful in practice in the field of clinical medicine.

Although a method for assaying a rheumatoid factor has been mainly described above, this fundamental technique is applicable for assaying glycoconjugates in general. The treating method for preparing the first reactant which can bind specifically to the glycoconjugate to be assayed, the sugar-chain-binding substance and the labeled compound may be appropriately selected in accordance with the description given above.

Examples of assays of glycoconjugates will now be described.

Infection with a bacterium or a virus can be diagnosed by the assay method according to the present invention since part of the surface proteins of the bacterium and the virus and antibodies against thereof are glycoproteins. Namely, the presence of a bacterium per se, i.e., an antigen, in the serum can be assayed by using a modified antibody prepared from an antibody against this bacterium as the modified first reactant and a lectin as the second reactant. On the contrary, the presence of an antibody against a bacterium in the serum may be detected by using a modified glycoprotein which has been prepared by modifying a constituting glycoprotein of the bacterium as the modified first reactant and a lectin as the second reactant.

To assay a glycoprotein which is a biological component, a modified antibody prepared from an antibody against the target substance, i.e., the glycoprotein, may be used as the modified first reactant. In particular, when the existence or inexistence or the amount of a glycoprotein closely relates to a certain disease pathologically, the application of the present invention to the detection of the glycoprotein is highly meaningful in pathology and clinical medicine. Examples of such glycoproteins include α-fetoprotein and β-galactosidase which relate to cancer, and the assay method according to the present invention is also applicable to a system for assaying such a glycoprotein.

There have also been known glycolipids of which existence or inexistence or amount closely relate to diseases. For example, it has been confirmed that antibodies (a kind of autoantibodies) which react with gangliosides such as GM1, GD1b and asialyl GM1 exist in the serum of a patient with Guillan-Barre syndrome which is one of nervous diseases [see Yuki, N., et al., Neurology, 40, 1900 (1990)]. It is thought that these antibodies cause nervous disorders including numbness, sensory disturbance and muscular weakness. The method of the present invention can be applied for assaying these antibodies.

Further, it is expected that the method of the present invention with the use of a sugar-chain-binding substance such as a lectin has another significance. Namely, when a glycoconjugate, which closely relates to a certain disease and is liberated into the blood during the disease may not be in the normal form but suffer from a change in its sugar chain moiety (for example, the sugar chain of α-fetoprotein in the serum of a patient with hepatoma differs from the normal one), it is possible that only one of a glycoconjugate liberated into the blood during the disease and a glycoconjugate of normal type can be discriminated by selecting an appropriate sugar-chain-binding substance as the second reactant. As the result, it is expected that novel clinical data and clinical images, which differ from those obtained by the conventional assay methods, can be obtained.

### Brief Description of the Drawing

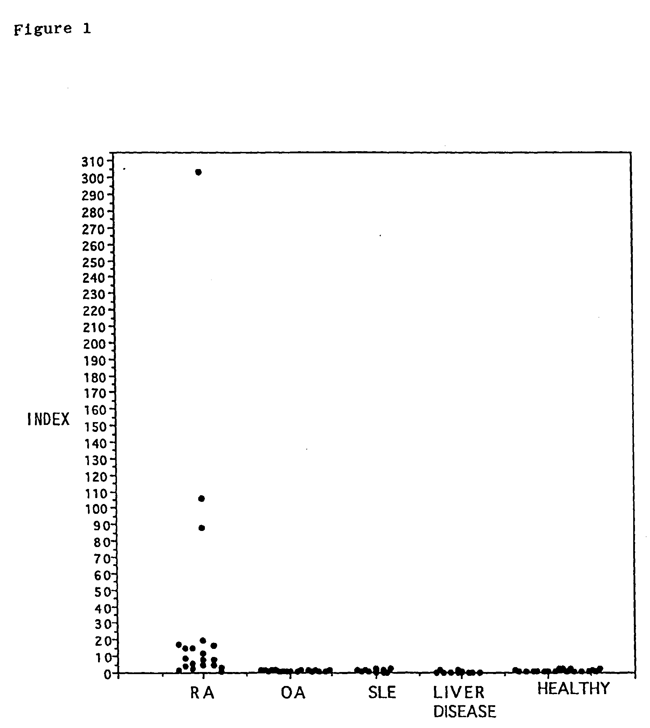

Fig. 1 shows the results of the assay of a rheumatoid factor in the serum specimens of various subjects effected in the above Example 4.

### Examples

The present invention will now be described in more detail with reference to the following Examples which should not be considered to limit the scope of the present invention.

### Example 1 Treatment of IgG with periodic acid

Metaperiodic acid was dissolved in PBS (50 mM phosphate buffer. 0.15 M NaCl, pH 7.4) and the resulting solution was added to a human IgG solution (1 mg/ml PBS) in such an amount as equivalent to that of the human IgG solution. Then, the mixture thus obtained was allowed to stand for 1 hour in the dark at room temperature to thereby perform oxidation. After the completion of the oxidation, the reaction mixture was dialyzed against PBS at 4°C to thereby give periodic acid-treated IgG. This metaperiodic acid-treated IgG will be referred to simply as LN-IgG (lectin-nonreactive IgG) hereinafter.

### Example 2 Immobilization of LN-IgG

A 5 µg/ml solution of LN-IgG in PBS was prepared and pipetted into wells of a polystyrene microplate in 100 µl portions. By allowing to stand the polystyrene microplate at 4°C over day and night, the LN-IgG was immobilized. After the immobilization, the solution of LN-IgG in PBS was sucked off. Then, 300 µl portions of 1% solution of BSA in PBS were added respectively to the wells and blocking at 4°C over day and night was effected. The polystyrene microplate thus treated was used in Example 4.

### Example 3 Reactivity of LN-IgG with anti-IgG antibody and lectins

2 µl portions of LN-IgG and normal IgG (each 1 mg/ml) were dot-blotted onto a nitrocellulose membrane. After drying, blocking with a gelatin buffer (0.25% gelatin, 0.05% NP-40, 0.15 M NaCl, pH 7.4) was effected at room temperature for 60 minutes. After the completion of the blocking, 500 µl of a peroxidase-labeled lectin (RCA120, Con A or LCA, 2.5 µg/ml) or peroxidase-labeled goat antihuman IgG antibody (prepared by diluting a marketed solution 500-fold) was added thereto. After the reaction at room temperature for 60 minutes and washing with a blocking buffer, 100 µl of a solution of 4-chloronaphthol [60 mg of 4-chloronaphthol, 20 µl of H₂O₂, 20 ml of methanol in 100 ml of 50 mM Tris-HCl buffer containing 0.5 M of NaCl (pH 7.4)], i.e., the substrate of peroxidase, was added thereto and the color development was observed.

As a result, it was confirmed that LN-IgG had reacted with the goat antihuman IgG antibody similar to the normal IgG, i.e., untreated IgG. This fact suggests that the antigenicity of the metaperiodic acid-treated IgG had not disappeared.

Regarding the reactivity with lectins (RCA120, Con A and LCA), it had been confirmed that the normal IgG reacted with these lectins while LN-IgG did not react-therewith (refer to Table 1).

Namely, it is suggested that the lectin recognition site of LN-IgG was blocked. Based on these results, it has been proved that LN-IgG has lost the reactivity with lectins but maintains its antigenicity.

**Table 1**

| Binding activity to lectins | | |
|---|---|---|
| | LN-IgG | Normal IgG |
| RCA120 | - | ++ |
| Con A | - | + |
| LCA | - | + |

### Example 4 Assay of a rheumatoid factor in serum specimens of patients and healthy subjects

Serum specimens of 20 patients with rheumatoid arthritis (RA), 18 with osteoarthritis (OA), 10 with systemic lupus erythematosus (SLE), 10 with liver disease and 20 healthy subjects were subjected to an assay of a rheumatoid factor.

### Assay method:

Each serum specimen was preliminarily diluted 200-fold with a gelatin buffer (2.5% gelatin, 0.15 M NaCl, 0.05% NP-40, 50 mM Tris-HCl, pH 7.4). 200 µl portions of the solution were added respectively to the wells of the LN-IgG immobilized polystyrene microplate prepared in accordance with the procedure of Example 2. After the reaction at 25°C for 60 minutes and washing, 100 µl portions of RCA120-biotin (2.5 µg/ml) were added respectively to the wells and the reaction was effected at 25°C for 60 minutes. After the completion of the reaction, these wells were washed and then 100 µl portions of streptoavidin-peroxidase (prepared by diluting a marketed product 1,000-fold) were added respectively thereto. After the reaction at 25°C for 60 minutes and washing, 100 µl portions of a solution of the substrate of peroxidase (6 mg ATBS, 10 µl H₂O₂ and 4 ml of 0.1 M citrate buffer, pH 4.3) were added respectively to the wells and the reaction was effected at 37°C for 30 minutes. After the completion of the reaction, 100 µl portions of NaN₃ were added respectively to the wells and the absorbances at two wavelengths of 405 nm and 490 nm were determined. Differences between the absorbances determined at 405 nm and those determined at 490 nm (A₄₀₅ -A₄₉₀) were calculated.

The cutoff value was set at a level thrice as high as the average of the specimens of healthy subjects (index 3) and data exceeding the cutoff value were judged as positive.

As is apparent from the results given in Fig. 1, the RA patients exclusively showed a positive ratio of 80% while no positive specimen was detected from the groups of the patients with OA, SLE and liver disease. Namely, this assay method was highly specific for the group of the RA patients.

The invention being thus described, it will be obvious that the same may be varied in many ways.

## Claims

1. A method for assaying a rheumatoid factor (RF) by the sandwich method which comprises:
1) reacting a periodic acid-treated immunoglobulin (IgG), which has been prepared by treating a sugar chain or an IgG having an avidity specific for a RF to thereby cause the loss of its avidity for a lectin, with a specimen to thereby bind the RF in the specimen thereto;
2) reacting said lectin having an avidity for the sugar chain of the RF as a second reactant with the RF thus bound; and
3) determining the amount of the lectin thus bound.

2. The method for assaying a RF as claimed in Claim 1, wherein said lectin is a labeled lectin.

3. The method for assaying a RF as claimed in Claim 1, wherein said lectin is labeled with a substance selected from the group consisting of enzymes, fluorescent substances, pigments, chemiluminescent substances, electroluminescent substances, metals and isotopes.

4. The method for assaying a RF as claimed in Claim 1, wherein a solid phase, on which said modified IgG is immobilized, is used.

5. The method for assaying a RF as claimed in Claim 1, wherein said lectin is one selected from the group consisting of Ricinus communis agglutinin (RCA120), concanavalin A (Con A) and Lens culinaris (LCA).

6. A kit for assaying a RF which comprises a periodic acid-treated IgG, which has been prepared by treating a sugar chain or an IgG having an avidity specific for a RF to thereby cause the loss of its avidity for a lectin, and a lectin as the essential constituents.

7. The kit for assaying a RF as claimed in Claim 6, wherein said lectin is one selected from the group consisting of Ricinus communis agglutinin (RCA120), concanavalin A (Con A) and Lens culinaris (LCA).

## Patentansprüche

1. Verfahren zur Untersuchung eines Rheumafaktors (RF) nach der Sandwichmethode durch:
(1) Einwirken eines Periodsäure-behandelten Immunglobulins (IgG), welches durch die Behandlung einer Zucker-Kette eines IgGs vorbereitet wurde, das eine spezifische Reaktionsfreudigkeit für einen RF hat, um dadurch den Verlust ihrer Reaktionsfreudigkeit für ein Lektin zu bewirken, auf eine Probe, um dadurch den RF in der Probe daran zu binden;
(2) Reagieren des Lektins mit einer Reaktionsfreudigkeit für die Zuckerkette des RF als zweite Reaktante mit dem so gebundenen RF; und
(3) Bestimmen der Menge des so gebundenen Lektins.

2. Verfahren zur Untersuchung eines RF gemäß Anspruch 1, wobei das Lektin ein markiertes Lektin ist.

3. Verfahren zur Untersuchung eines RF gemäß Anspruch 1, wobei das Lektin mit einer aus der Gruppe bestehend aus Enzymen, fluoreszierenden Substanzen, Pigmenten, chemilumineszenten Substanzen, elektrolumineszenten Substanzen, Metallen und Isotopen ausgewählten Substanz markiert ist.

4. Verfahren zur Untersuchung eines RF gemäß Anspruch 1, wobei eine Festphase verwendet wird, auf der das modifizierte IgG immobilisiert ist.

5. Verfahren zur Untersuchung eines RF gemäß Anspruch 1, wobei das Lektin aus der aus Rhizinus - communis - Agglutinin (RCA120), Concanavalin A (Con A) und Lens culinaris (LCA) bestehenden Gruppe ausgewählt ist.

6. Kit zur Untersuchung eines RF, mit einem Periodsäurebehandelten IgG, das durch die Behandlung einer Zuckerkette eines IgG, das eine spezifische Reaktionsfreudigkeit für einen RF hat, um dadurch den Verlust seiner Reaktionsfreudigkeit für ein Lektin zu bewirken, vorbereitet wurde und einem Lektin als wesentliche Bestandteile.

7. Kit zur Untersuchung eines RF gemäß Anspruch 6, wobei das Lektin aus der aus Rhizinus - communis - Agglutinin (RCA120), Concanavalin A (Con A) und Lens culinaris (LCA) bestehenden Gruppe ausgewählt ist.

## Revendications

1. Procédé pour la détermination d'un facteur rhumatoïde (RF) par la méthode en sandwich, qui comprend:
1) la réaction d'une immunoglobuline (IgG) traitée par l'acide periodique, qui a été préparée par traitement d'une chaîne sucre d'une IgG ayant une avidité spécifique pour un RF pour provoquer ainsi la perte de son avidité pour une lectine, avec un spécimen pour y fixer ainsi le RF dans le spécimen ;
2) la réaction de ladite lectine ayant une avidité pour la chaîne sucre du RF en tant que deuxième réactif avec le RF ainsi fixé; et
3) la détermination de la quantité de la lectine ainsi fixée.

2. Procédé pour la détermination d'un RF selon la revendication 1, dans lequel ladite lectine est une lectine marquée.

3. Procédé pour la détermination d'un RF selon la revendication 1, dans lequel ladite lectine est marquée avec une substance choisie dans le groupe consistant en des enzymes, des substances fluorescentes, des pigments, des substances chimioluminescentes, des substances électroluminescentes, des métaux et des isotopes.

4. Procédé pour la détermination d'un RF selon la revendication 1, dans lequel une phase solide, sur laquelle ladite IgG est immobilisée, est utilisée.

5. Procédé pour la détermination d'un RF selon la revendication 1, dans lequel ladite lectine est une lectine choisie dans le groupe consistant en agglutininine de Ricinus communis (RCA120), concanavaline A (Con A) et Lens culinaris (LCA).

6. Kit ou ensemble pour la détermination d'un RF, qui comprend une IgG traitée par l'acide periodique, qui a été préparée par traitement d'une chaîne sucre d'une IgG ayant une avidité spécifique pour un RF pour y provoquer ainsi la perte de son avidité pour une lectine, et une lectine en tant que constituants essentiels.

7. Kit pour la détermination d'un RF selon la revendication 6, dans lequel ladite lectine est une lectine choisie dans le groupe consistant en agglutininine de Ricinus communis (RCA120), concanavaline A (Con A) et Lens culinaris (LCA).
